# EUROPEAN PATENT APPLICATION

(11) **EP 4 623 939 A1**
(43) Date of publication of application: **01.10.2025**
(21) Application number: 25166559.2
(22) Date of filing: 27.03.2025
(51) Int. Cl.: A61L 2/04, A61L 2/24, A61L 2/18, E05F 15/00

(54) **TREATMENT APPARATUS WITH TECHNICAL COMPARTMENT AND CORRESPONDING METHOD FOR ACCESSING SUCH COMPARTMENT**

(30) Priority: 28.03.2024 IT 202400006991
(71) Applicant: Steelco S.p.A., 31039 Riese Pio X (IT)
(72) Inventor: Dall'Armi, Eriberto, 31041 Cornuda (TV) (IT); Brancaleon, Marco, 31039 Riese Pio X (TV) (IT); Prandoni Kistner, Andre, 36040 Torri di Quartesolo (VI) (IT)
(74) Representative: Petraz, Gilberto Luigi

(57) **Abstract**

Apparatus (10) for treating objects comprising a treatment chamber (11), an adjacent technical compartment (12) and a safety control unit (26), and corresponding method for accessing the technical compartment (12).

## Description

### FIELD OF THE INVENTION

The present invention concerns an apparatus for treating, in particular for washing, thermally disinfecting and/or sterilizing, objects for sanitary, medical or veterinary use. The objects can be, for example, but not limited to, trolley-mounted objects such as hospital beds, trolleys or other sanitary furnishing elements, or instruments, devices or containers for surgical, medical or laboratory use. The present invention also concerns a method for an operator to access a technical compartment of this treatment apparatus, for example in order to manage at least one treatment fluid tank present in the technical compartment and/or for maintenance and/or repair interventions in the technical compartment.

### BACKGROUND OF THE INVENTION

Washing, thermal disinfection and/or sterilization apparatuses for objects used in the health, medical and veterinary sectors and in research laboratories, and therefore subject to contamination, are known. Some examples of objects to be subjected to a washing, thermal disinfection and/or sterilization treatment are containing devices for laboratory animals, operating theatre instruments, laboratory instruments, hospital furniture, for example hospital beds, or trolleys. Known apparatuses are provided with a treatment chamber inside which the objects to be treated are introduced on each occasion, typically supported by trolley-mounted transport means. A system for delivering treatment fluids, such as liquids or steam to carry out a desired treatment cycle, is provided in the treatment chamber.

For this purpose, a hydraulic distribution unit for distributing the treatment substance is typically provided, provided with a pump and pipes that carry the treatment substance into the treatment chamber. The treatment substance can come from tanks housed inside a technical compartment adjacent to, but separated from, the treatment chamber.

The sizes of the technical compartment are generally small, but such as to allow access by an operator to allow for maintenance interventions on the apparatus. The technical compartment typically has a front access door, usually adjacent to an access door to the aforementioned treatment chamber, so as to allow the replacement or replenishing of the treatment substance tanks and access to the technical compartment for maintenance interventions.

For safety reasons, the entry of an operator into the technical compartment can only occur in conditions wherein the apparatus is blocked. Generally, for safety reasons, the opening of the access door to the technical compartment places the apparatus in a state of alarm that can only be resolved (thus restoring the apparatus' normal operation) by an authorized technician, and therefore not by a general user of the apparatus.

This leads to the disadvantage of having to request the intervention of an authorized technician in order to restore the normal operation of the apparatus even in the event that the access door to the technical compartment is opened only to replace or replenish the treatment substance tanks, an operation that does not require the operator to enter the technical compartment.

Another disadvantage is represented by the overall dimensions of the treatment substance tanks, which have to be completely removed from the technical compartment in case of maintenance, both to allow access and also to guarantee the presence of an escape route in case of emergency.

There is therefore the need to perfect a treatment apparatus that can overcome at least one of the disadvantages of the state of the art.

To do this, it is necessary to resolve the technical problem of positioning treatment substance tanks in the technical compartment in a compact and easily accessible manner, while at the same time guaranteeing the safety and practicality of a potential entry of an operator into the technical compartment.

In particular, one purpose of the present invention is to provide an apparatus for treating objects that allows entry into the technical compartment only in conditions wherein the apparatus is blocked, and the treatment substance tanks are removed from emergency escape routes.

Another purpose of the present invention is to provide an apparatus for treating objects that allows easy access to the treatment substance tanks for removal, addition, replacement and/or filling.

Another purpose of the present invention is to perfect methods of safe access to the technical compartment to enter the technical compartment and manage the treatment substance tanks.

The Applicant has devised, tested and embodied the present invention to overcome the shortcomings of the state of the art and to obtain these and other purposes and advantages.

### SUMMARY OF THE INVENTION

The present invention is set forth and characterized in the independent claims. The dependent claims describe other characteristics of the present invention or variants to the main inventive idea.

In accordance with the above purposes and to resolve the technical problem described above in a new and original way, also achieving considerable advantages compared to the state of the prior art, some embodiments described here concern an apparatus for treating, understood as washing, thermally disinfecting and/or sterilizing, objects which comprises a chamber for treating such objects, an adjacent technical compartment accessible via an aperture of the compartment with a closing assembly and a safety control unit.

In some embodiments, the closing assembly comprises a first and second door configured to selectively close a respective first and second portion of the compartment's aperture. A trolley configured to support a tank for a treatment substance can be positioned inside the technical compartment. The trolley is configured as mobile to selectively assume an extended position in which it obstructs the aperture's first portion and a retracted position in which it does not obstruct it. The apparatus comprises at least one clamping unit configured to engage the trolley in the retracted position and able to transmit a signal to the safety control unit correlated to the trolley's extended or retracted position. The closing assembly comprises a closing sensor assembly configured to transmit a signal to the security control unit correlated to a closed or open status of the second door. The safety control unit is configured to inhibit the operation of the apparatus when the clamping unit transmits a signal correlated to the trolley's retracted position and/or the closing sensor assembly transmits a signal correlated to the second door's open status.

By doing so, it is advantageously possible to position treatment substance tanks in the technical compartment in a compact and easily accessible manner, while at the same time guaranteeing the safety and practicality of any potential entry of an operator into the technical compartment, allowing entry into the technical compartment only in conditions wherein the apparatus is blocked, and the tanks are removed from any emergency escape routes.

Other embodiments described here concern a method for accessing the technical compartment of the apparatus described above. This method can provide to make available an apparatus as described above, with the trolley in an extended position and both the first and second door closed, and to open at least the first door, thus making the at least one tank accessible to an operator. The trolley is in an extended position; thus it overlaps with the first portion of the technical compartment's aperture and obstructs it.

If it is necessary to manage, that is, remove, add, replace and/or fill the at least one tank, the method can provide to manage the at least one tank and then, optionally, close the first door.

If it is necessary for an operator to enter the technical compartment for maintenance reasons, the method can provide that:
- after the opening of the first door, the trolley is moved into the retracted position and the clamping unit transmits a signal to the safety control unit correlated to the retracted position. The clamping unit can engage the trolley in the retracted position;
- the safety control unit inhibits the operation of the apparatus;
- before or after the opening of the first door and/or the movement of the trolley into the retracted position, the second door is opened. The closing sensor assembly transmits a signal correlated to this open status to the safety control unit, which inhibits the operation of the apparatus;
- an operator enters the technical compartment and carries out the necessary maintenance operations on the apparatus inside it.

### DESCRIPTION OF THE DRAWINGS

These and other aspects, characteristics and advantages of the present invention will become apparent from the following description of some embodiments, given as a non-restrictive example with reference to the attached drawings wherein:
- fig. 1 is a perspective view of a washing, thermal disinfection and/or sterilization apparatus with a technical compartment according to the present invention, with external wall portions removed in order to show the technical compartment's interior;
- figs. 2-4 are rear views of the technical compartment of fig. 1, without an external wall in order to show the technical compartment's interior, with a trolley of the technical compartment in an extended (figs. 2, 3) or retracted (fig. 4) position.

We must clarify that the phraseology and terminology used in the present description, as well as the figures in the attached drawings also in relation as to how described, have the sole function of better illustrating and explaining the present invention, their purpose being to provide a non-limiting example of the invention itself, since the scope of protection is defined by the claims.

To facilitate comprehension, the same reference numbers have been used, where possible, to identify identical common elements in the drawings. It is understood that elements and characteristics of one embodiment can be conveniently combined or incorporated into other embodiments without further clarifications.

### DESCRIPTION OF SOME EMBODIMENTS

A treatment apparatus 10 according to the present invention can be used to subject objects affected by contamination, such as laboratory animal containing devices, operating theatre instruments, laboratory instruments, hospital furniture, for example hospital beds, or trolleys, to a washing, thermal disinfection and/or sterilization treatment.

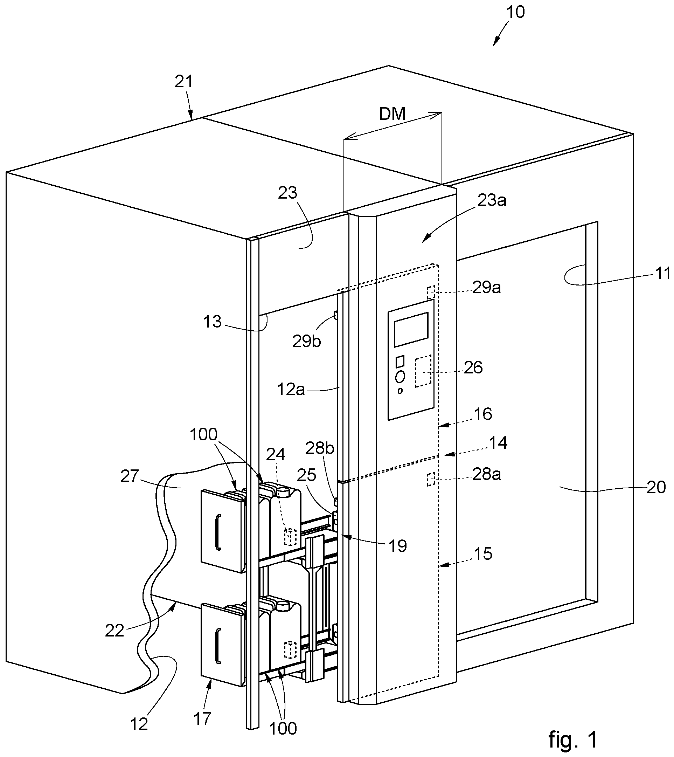

With reference to fig. 1, the apparatus 10 comprises a treatment chamber 11 for treating the objects and an adjacent technical compartment 12.

The apparatus 10 can be delimited by a plurality of external walls, for example forming a first box-shaped body 21 with a parallelepiped shape with a quadrangular, rectangular or square base.

An internal volume of the first box-shaped body 21 can be divided by one or more internal walls into the treatment chamber 11 and the technical compartment 12. In some embodiments, the treatment chamber 11 can be delimited by a plurality of internal and/or external walls, for example forming a second box-shaped body 22 with a parallelepiped shape with a quadrangular, rectangular or square base. The treatment chamber 11 can be accessible from at least one access door 20.

The treatment chamber 11 can comprise a system for delivering and/or introducing at least one treatment substance. By treatment substance we mean a substance that can be used to carry out all or part of a desired treatment cycle of objects, such as washing, thermal disinfection and/or sterilization. The at least one treatment substance can come from at least one tank 100.

In the embodiments shown in figs. 1-4, the technical compartment 12 is defined by the internal volume of the first box-shaped body 21 and by the volume external to the second box-shaped body 22. In some embodiments, not shown in the attached drawings, the technical compartment 12 can be defined and delimited by a plurality of walls forming another box-shaped body that is internal to the first box-shaped body 21 and adjacent to the second box-shaped body 22.

The treatment chamber 11 and the technical compartment 12 can have a common wall 27 shared between them.

The technical compartment 12 can be able to contain the at least one or each tank 100 for a treatment substance. The, or each, tank 100 can be connected to the delivery and/or introduction system by means of suitable pumps and circuits, so that the at least one treatment substance can be delivered into the treatment chamber 11 through the delivery and/or introduction system thereof.

The technical compartment 12 can be accessible via an aperture 13 of the technical compartment 12. The aperture 13 can be created in a portion of a wall of the technical compartment 12, for example in a portion of a wall of the first box-shaped body 21, or external wall 23.

Preferably, the aperture 13 is created in a portion of the external wall 23 distanced from the treatment chamber 11 by a minimum distance DM, thus defining a portion 12a of the technical compartment 12 which is interposed between the treatment chamber 11 and the aperture 13, hereafter referred to as interposed portion 12a for short, and a corresponding external wall portion 23a of the external wall 23 (figs. 1-4).

The sizes of the aperture 13 are preferably suitable to allow an operator to enter the technical compartment 12.

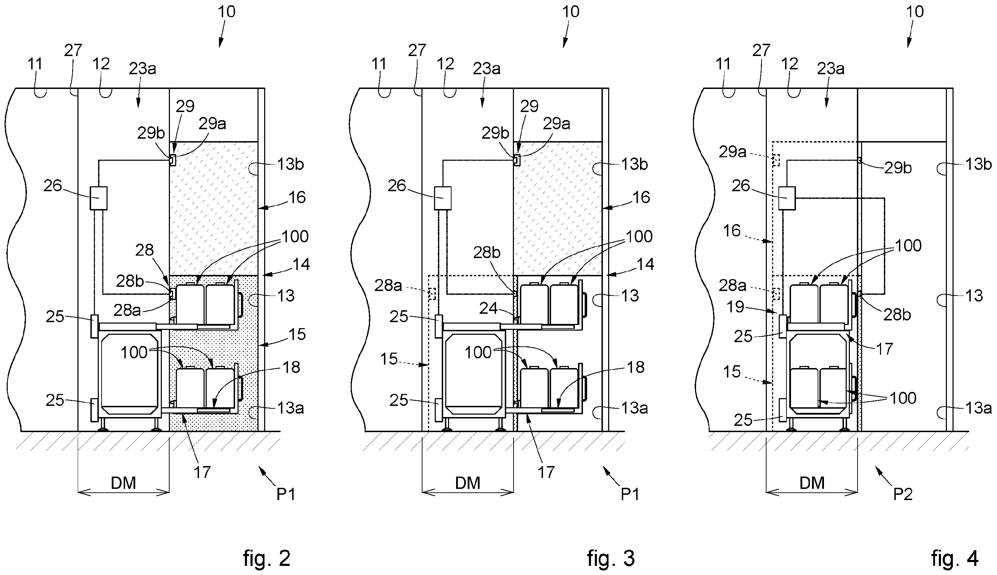

A trolley 17 can be positioned inside the technical compartment 12, in association with the aperture 13. The trolley 17 can be configured to support the at least one tank 100. The trolley 17 can comprise at least one support area 18 for tanks 100 (fig. 2). The at least one or each tank 100 can be installed on the support area 18.

The trolley 17 can be configured as mobile to selectively assume an extended position P1 of partial obstruction of the aperture 13 (figs. 1-3) and a retracted position P2 of clearing of the aperture 13 (fig. 4). In the extended position P1, the support area 18 can be aligned with the aperture 13 in such a way that every tank 100 possibly installed on the support area 18 is accessible from the outside. By "accessible from the outside" we mean that the tank 100 can be removed, replaced and/or filled from the outside. Moreover, a new tank 100 can be added onto the support area 18.

The overall dimension of the trolley 17 in the extended position P1 and of all the tanks 100 that can be installed on the trolley 17 defines a first portion 13a obstructed by this overall dimension and a second portion 13b of the aperture 13 not obstructed by this overall dimension (figs. 2-4).

The first portion 13a and the second portion 13b can be, for example, a lower portion and an upper portion of the aperture 13, respectively.

Preferably, the sizes of the first and second portion 13a, 13b are such as to not allow, if taken individually, an operator to enter into the technical compartment 12.

The trolley 17 can be configured as mobile to selectively assume the extended position P1, in which it obstructs the first portion 13a, since the support area 18 overlaps with the first portion 13a, and the retracted position P2, in which the trolley 17 does not obstruct the first portion 13a, since it does not overlap with the first portion 13a.

The trolley 17 can be configured to obstruct, in the extended position P1, the first portion 13a in such a way as to prevent an operator from entering into the technical compartment 12 (figs. 1-3).

In the retracted position P2, the trolley 17 can be completely removed from the first portion 13a and retracted toward the treatment chamber 11 (fig. 4). The trolley 17 can be configured to leave, in the retracted position P2, the first portion 13a free in such a way as to allow an operator to enter into the technical compartment 12. The trolley 17 can be configured to be completely contained, in the retracted position P2, in the interposed portion 12a of the technical compartment 12.

The apparatus 10 can comprise at least one clamping unit 19 (figs. 1, 4) configured to engage the trolley 17 in the retracted position P2. The clamping unit 19 can be configured to block or clamp the trolley 17 in the retracted position P2. For example, the clamping unit 19 can comprise a protruding element 24 integral with the trolley 17 (figs. 1-3).

The protruding element 24 can be positioned on the trolley 17 so as to face the treatment chamber 11. For example, the protruding element 24 can face the common wall 27.

The clamping unit 19 can also comprise a clamping member 25 configured to accommodate and clamp the protruding element 24 in place. The clamping member 25 can be installed in correspondence with or in proximity to the common wall 27, facing the protruding element 24.

The clamping unit 19 can be configured in such a way that, in the retracted position P2, the protruding element 24 is inserted into the clamping member 25 in such a way that the latter clamps it in position, thus in fact clamping the entire trolley 17 in the retracted position P2 (fig. 4).

The clamping unit 19 can comprise an electric lock.

The aperture 13 can be closed by a closing assembly 14 (figs. 1, 2). The closing assembly 14 can comprise a first door 15, for example a lower door, and a second door 16, for example an upper door (figs. 1-4). The first door 15 and the second door 16 can be configured to selectively close, as a whole, the entire aperture 13 (fig. 2). The first and second door 15, 16 can be configured to selectively close the first and second portion 13a, 13b of the aperture 13, respectively.

For example, the first and second door 15, 16 can be sliding doors, configured to slide from a closed status to an open status, or vice versa. In the respective closed statuses, the first and second door 15, 16 engage the respective first or second portion 13a, 13b of the aperture 13 (fig. 2). In the respective open statuses, the first and second door 15, 16 are positioned in the interposed portion 12a, for example in a special hollow space of the external wall portion 23a (figs. 1, 4).

The closing assembly 14 can comprise a closing sensor assembly 29 of the second door 16 (fig. 2), configured to detect a closed or open status of the second door 16.

For example, the closing sensor assembly 29 can comprise a respective magnet 29a and a respective magnetic sensor 29b. The magnet 29a can be positioned on the second door 16, for example in such a way that, when the second door 16 is closed, the magnet 29a is on the side of the second door 16 closest to the external wall portion 23a. The magnetic sensor 29b can be positioned on the perimeter frame of the aperture 13, for example on the side of the external wall portion 23a facing the second portion 13b of the aperture 13. The closing sensor assembly 29 can be configured in such a way that, when the second door 16 is closed, the magnet 29a and the magnetic sensor 29b face each other.

The closing assembly 14 can comprise another closing sensor assembly 28 of the first door 15 (fig. 2), configured to detect a closed or open status of the first door 15. For example, the other closing sensor assembly 28 can comprise a respective other magnet 28a and a respective other magnetic sensor 28b. The other magnet 28a can be positioned on the first door 15, for example in such a way that, when the first door 15 is closed, the other magnet 28a is on the side of the first door 15 closest to the external wall portion 23a.

The other magnetic sensor 28b can be positioned on a perimeter frame of the aperture 13, for example on a side of the external wall portion 23a facing the first portion 13a of the aperture 13. The other closing sensor assembly 28 can be configured in such a way that, when the first door 15 is closed, the other magnet 28a and the other magnetic sensor 28b face each other.

The apparatus 10 can comprise a safety control unit 26, configured to selectively inhibit the operation of the apparatus 10. The safety control unit 26 can be configured to selectively generate an alarm signal able to inhibit the operation of the apparatus 10. The alarm signal can cause a configuration of the apparatus 10 to change from an operating configuration to an alarm, or blocked, configuration. In the operating configuration, the apparatus 10 can perform a desired treatment cycle in the treatment chamber 11. In the alarm, or blocked, configuration, the apparatus 10 cannot carry out any treatment cycles. Preferably, for safety reasons, the safety control unit 26 is configured to generate the alarm signal, thus inhibiting the operation of the apparatus 10, when the second door 16 is open. Preferably, for safety reasons, the operation of the apparatus 10 in the alarm configuration can only be restored by an authorized technician.

The closing sensor assembly 29 can be configured to transmit a signal to the safety control unit 26 correlated to a closed or open status of the second door 16. For example, the closing sensor assembly 29 can be configured to transmit to the safety control unit 26 a closed status signal as long as the second door 16 is closed and/or an open status signal as long as the second door 16 is open. The safety control unit 26 can be configured to inhibit the operation of the apparatus 10, for example by generating the alarm signal, when the closing sensor assembly 29 transmits a signal correlated to the open status of the second door 16, that is, when the closing sensor assembly 29 transmits the open status signal and/or interrupts the closed status signal.

The safety control unit 26 can also be configured to selectively enable or disable the opening of the, or of each, access door 20 to the treatment chamber 11.

The other closing sensor assembly 28 can be configured to transmit a signal correlated to the closed or open status of the first door 15 to the safety control unit 26. For example, the other closing sensor assembly 28 can be configured to transmit a closed status signal to the safety control unit 26 as long as the first door 15 is closed. Alternatively or additionally, the other closing sensor assembly 28 can be configured to transmit an open status signal to the safety control unit 26 as long as the first door 15 is open. The safety control unit 26 can be configured to disable the opening of the, or of each, access door 20 to the treatment chamber 11 when the other closing sensor assembly 28 transmits a signal correlated to the open status of the first door 15, that is, when it transmits the open status signal and/or interrupts the closed status signal.

Furthermore, the clamping unit 19 can be able to transmit a signal to the safety control unit 26 correlated to the extended P1 or retracted P2 position of the trolley 17. The clamping unit 19 can be configured to transmit an extended status signal to the safety control unit 26 as long as the trolley 17 is in the extended position P1. Alternatively or additionally, the clamping unit 19 can be configured to transmit a retracted status signal to the safety control unit 26 as long as the trolley 17 is in the retracted position P2.

In some embodiments, the clamping unit 19 is configured to determine whether the trolley 17 is in the extended P1 or retracted P2 position based on the unclamping or engagement status of the protruding element 24 and of the clamping member 25. In some embodiments, the clamping unit 19 comprises a position sensor assembly, for example a magnetic sensor, configured to determine whether the trolley 17 is in the extended P1 or retracted P2 position.

The safety control unit 26 can be configured to inhibit the operation of the apparatus 10, for example by generating the alarm signal, when the clamping unit 19 transmits a signal correlated to the retracted position P2, that is, when it transmits the signal of retracted status from the clamping unit 19 and/or when it interrupts the signal of extended status from the clamping unit 19.

In some embodiments, the apparatus 10 can comprise a device for electronically or electromechanically clamping the second door 16. This clamping device can be configured to keep the second door 16 clamped in the closed position when the first door 15 is in the closed position and/or when the trolley 17 is in the extended position P1.

The clamping device can be configured to release the second door 16, allowing it to be opened, when the first door 15 is in the open position and the trolley 17 is in the retracted position P2.

The operation of the apparatus 10 described heretofore comprises a method for accessing the technical compartment 12, in order to manage the tanks 100 and/or enter for maintenance.

This method can comprise:
- making the apparatus 10 available in the operating configuration, with the trolley 17 in the extended position P1 and the first and second door 15, 16 both closed (fig. 2);
- opening at least the first door 15 (fig. 3).

The opening of the first door 15 makes each tank 100 present on the trolley 17 accessible to an operator. The trolley 17 is in the extended position P1, therefore it overlaps with the first portion 13a, thus obstructing it. The apparatus 10 can therefore remain in the operating configuration, since the presence of the trolley 17 physically blocks the entry of an operator into the technical compartment 12.

If present, the other closing sensor assembly 28 can transmit a signal correlated to the open status of the first door 15 (for example, interrupt the closed status signal and/or transmit the open status signal) to the safety control unit 26, which can consequently disable the opening of the access door 20.

The method for accessing the technical compartment 12 to manage the tanks 100 can therefore provide to manage, that is, remove, add, replace and/or fill the at least one tank 100.

Optionally, the first door 15 can be closed once management of the tanks has ended (fig. 2). If present, the other closing sensor assembly 28 can transmit a signal correlated to this closed status (for example, transmit the closed status signal and/or interrupt the open status signal) to the safety control unit 26, which can consequently enable the opening of the access door 20.

In order to access the technical compartment 12 so as to enter for maintenance, after the aforementioned opening of the first door 15, the trolley 17 is moved to the retracted position P2 (fig. 4). The movement can be done manually or in an automated manner. The clamping unit 19 can transmit a signal to the safety control unit 26 correlated to the retracted position P2 (for example, transmit the retracted status signal and/or interrupt the extended status signal).

When the trolley 17 reaches the retracted position P2, the clamping unit 19 can engage, and preferably clamp, the trolley 17. For example, the protruding element 24 is inserted into the clamping member 25, triggering the clamping, or blocking, in place of the protruding element 24 in the clamping member 25. The safety control unit 26 can then inhibit the operation of the apparatus 10, for example by generating the alarm signal. In other words, the apparatus 10 is put, or kept, in an alarm configuration.

Before or after the first door 15 is opened and/or the trolley 17 is moved into the retracted position P2, the second door 16 can be opened (fig. 4). The opening can be automatic or actively triggered by the user. The closing sensor assembly 29 can transmit a signal to the safety control unit 26 correlated to this open status. For example, the closing assembly 29 can interrupt the closed status signal and/or transmit the open status signal to the safety control unit 26. Consequently, the safety control unit 26 inhibits the operation of the apparatus 10, for example by generating the alarm signal, therefore the apparatus 10 is put, or kept, in an alarm configuration.

When the open status signal is received from the closing sensor assembly 29, the safety control unit 26 can transmit a clamping signal to the clamping unit 19, which consequently can clamp the trolley 17 in the retracted position P2.

The method can then provide that an operator enters the technical compartment 12 and carries out the necessary maintenance operations of the apparatus 10 inside the technical compartment 12.

At the end of the maintenance operations, the operator can exit the technical compartment 12 and the second door 16 can be closed. The closing sensor assembly 29 transmits a signal to the safety control unit 26 correlated to this closed status. For example, the closing sensor assembly 29 can transmit the closed status signal and/or interrupt the open status signal. The safety control unit 26 can then transmit an unclamping signal to the clamping unit 19, which can unclamp the trolley 17 from the retracted position P2. The apparatus 10 remains in an alarm configuration.

The method then comprises moving the trolley 17 into the extended position P1 (fig. 3). The movement can be done manually or in an automated manner. The clamping unit 19 can transmit a signal to the safety control unit 26 correlated to this extended position P1. For example, the clamping unit 19 can transmit the extended status signal and/or interrupt the retracted status signal. In those embodiments that provide it, the safety control unit 26 can activate the clamping device of the second door 16.

Subsequently, the method comprises restoring the operation of the apparatus 10, for example the interruption of the alarm signal by an authorized technician. In other words, the authorized technician restores the operating configuration of the apparatus 10.

Furthermore, it is optionally possible to close the first door 15 (fig. 2). If present, the other closing sensor assembly 28 can transmit a signal to the safety control unit 26 correlated to this closed status. For example, the other closing sensor assembly 28 can transmit the closed status signal and/or interrupt the open status signal to the safety control unit 26, which can consequently enable the opening of the access door 20.

It is clear that modifications and/or additions of parts may be made to the apparatus 10 and to the methods as described heretofore, without thereby departing from the field and scope of the present invention, as defined by the claims.

It is also clear that, although the present invention has been described with reference to some specific examples, a person of skill in the art will be able to achieve other equivalent forms of washing, thermal disinfection and/or sterilization apparatus with technical compartment, and corresponding methods, all having the characteristics as set forth in the claims and hence all coming within the field of protection defined thereby.

In the following claims, the sole purpose of the references in brackets is to facilitate their reading and they must not be considered as restrictive factors with regard to the field of protection defined thereby.

## Claims

1. Apparatus (10) for treating objects comprising a treatment chamber (11), an adjacent technical compartment (12) accessible via an aperture (13) with a closing assembly (14) and a safety control unit (26), **characterized in that:**
- said closing assembly (14) comprises a first and second (15, 16) door to selectively close a respective first or second (13a, 13b) portion of said aperture (13);
- a trolley (17) configured to support a tank (100) is positioned inside said technical compartment (12);
- said trolley (17) is configured as mobile to selectively assume an extended position (P1) in which it obstructs said first portion (13a) and a retracted position (P2) in which it does not obstruct said first portion (13a);
- said apparatus (10) comprises at least one clamping unit (19) configured to engage said trolley (17) in said retracted position (P2) and able to transmit a signal to said safety control unit (26) correlated to the extended or retracted position (P1, P2) of said trolley;
- said closing assembly (14) comprises a closing sensor assembly (29) configured to transmit a signal to said security control unit (26) correlated to a closed or open status of said second door (16),
wherein said safety control unit (26) is configured to inhibit the operation of said apparatus (10) when said clamping unit (19) transmits a signal correlated to said retracted position (P2) and/or said closing sensor assembly (29) transmits a signal correlated to said open status.

2. Apparatus (10) as in claim 1, **characterized in that** said clamping unit (19) is configured to clamp said trolley (17) in said retracted position (P2).

3. Apparatus (10) as in claim 1 or 2, **characterized in that** said clamping unit (19) comprises an electric lock.

4. Apparatus (10) as in any claim hereinbefore, **characterized in that** said closing sensor assembly (29) comprises a magnet (29a) positioned on said second door (16) and a magnetic sensor (29b) positioned on a perimeter frame of said aperture (13), said closing sensor assembly (29) being configured in such a way that, when said second door (16) is closed, said magnet and sensor (29a, 28b) face each other.

5. Apparatus (10) as in any claim hereinbefore, **characterized in that** said closing assembly (14) comprises another closing sensor assembly (28) of said first door (15) configured to transmit a signal to said security control unit (26) correlated to a closed or open status of said first door (15), said safety control unit (26) being configured to disable the opening of one or each access door (20) to said treatment chamber (11) when said other closing sensor assembly (28) transmits a signal correlated to said open status.

6. Apparatus (10) as in claim 5, **characterized in that** said other closing sensor assembly (28) comprises another magnet (28a) positioned on said first door (15) and another magnetic sensor (28b) positioned on a perimeter frame of said aperture (13), said other closing sensor assembly (28) being configured in such a way that, when said first door (15) is closed, said other magnet and sensor (28a, 28b) face each other.

7. Method for accessing a technical compartment (12) of an apparatus (10) for treating objects, **characterized in that** it comprises:
- making available an apparatus (10) as in any claim hereinbefore, with said trolley (17) in said extended position (P1) and both said first and second doors (15, 16) closed;
- opening at least said first door (15), thus making each tank (100) present on said trolley (17) in said technical compartment (12) accessible to an operator, said trolley (17) being overlapping with said first portion (13a), thus obstructing it.

8. Method as in claim 7, **characterized in that** it comprises, after said opening of said first door (15), managing, that is, removing, adding, replacing and/or filling, said at least one tank (100) and, optionally, closing said first door (15).

9. Access method as in claim 8, **characterized in that:**
- when said first door (15) is opened, another closing sensor assembly (28) transmits a signal correlated to the open status to said security control unit (26) which disables the opening of one or each access door (20) to said treatment chamber (11);
- when said first door (15) is closed, said other closing sensor assembly (28) transmits a signal correlated to the closed status to said safety control unit (26) which enables the opening of said access doors (20).

10. Access method as in claim 7, **characterized in that:**
- after said opening of said first door (15), said trolley (17) is moved into said retracted position (P2), said clamping unit (19) transmits a signal to said safety control unit (26) correlated to said retracted position (P2) and engages said trolley (17) in said retracted position (P2);
- said safety control unit (26) inhibits the operation of said apparatus (10);
- before or after the opening of said first door (15) and/or the movement of said trolley (17) into said retracted position (P2), said second door (16) opens, said closing sensor assembly (29) transmits a signal correlated to said open status to said safety control unit (26) which inhibits the operation of said apparatus (10);
- an operator enters said technical compartment (12) and carries out maintenance operations on said apparatus (10).

11. Access method as in claim 10, **characterized in that:**
- when said signal correlated to said open status is received from said closing sensor assembly (29), said safety control unit (26) transmits a clamping signal to said clamping unit (19) which consequently clamps said trolley (17) in said retracted position (P2);
- at the end of the maintenance operations in said technical compartment (12), said operator exits said technical compartment (12) and said second door (16) is closed, said closing sensor assembly (29) transmits a signal correlated to said closed status to said safety control unit (26) which transmits an unclamping signal to said clamping unit (19), which unclamps said trolley (17) from said retracted position (P2);
- said method comprises moving said trolley (17) into said extended position (P1) and said clamping unit (19) transmits a signal to said safety control unit (26) correlated to said extended position (P1);
- subsequently, said method comprises restoring the operation of said apparatus (10).
